# EUROPEAN PATENT SPECIFICATION

(11) **EP 2 502 172 B1**
(45) Date of publication and mention of the grant of the patent: **25.12.2019**
(21) Application number: 10787999.1
(22) Date of filing: 13.11.2010
(51) Int. Cl.: G16H 20/17, G16H 40/63

(54) **HYPOGLYCEMIC TREATMENT SYSTEMS**
BEHANDLUNGSSYSTEM FÜR HYPOGLYKÄMIE
SYSTÈMES POUR TRAITEMENT DE L'HYPOGLYCÉMIE

(30) Priority: 17.11.2009 US 620202
(43) Date of publication of application: 26.09.2012
(73) Proprietor: Roche Diabetes Care GmbH, 68305 Mannheim (DE); F. Hoffmann-La Roche AG, 4070 Basel (CH)
(72) Inventor: TIMMERMAN, Robert David, Fishers IN 46038 (US)
(74) Representative: Bittner, Thomas L.
(86) International application number: PCT/EP2010/006924
(87) International publication number: WO 2011/060908

(56) References cited:
- WO-A1-2009/048462
- WO-A2-2008/011389
- US-A1- 2003 125 612
- US-B1- 6 572 542

## Description

### Technical Field

The present invention relates generally to hypoglycemic treatment methods and systems.

### Background

Hypoglycemic or hypoglycemia refers to a condition of a lower than normal level of blood glucose. Hypoglycemia may occur, for example, as a complication of treatment of diabetes. Hypoglycemia, although less common, may also occur in non-diabetic persons and may occur at any age.

A diabetic can become hypoglycemic at any time of day. More severe complications due to hypoglycemia include seizures, coma, brain damage or even death. It is desirable to provide hypoglycemic treatment systems and methods capable of alerting and/or treating hypoglycemia.

Document WO 2008/011389 A2 discloses ambulatory medical systems that include a monitoring device, a therapeutic substance administering device, and a receiver-controller for receiving a signal generated by the monitoring device and controlling the therapeutic substance administering device based on the information received from the monitoring device.

Document WO 2009/048462 A1 pertains to systems and methods for integrating a continuous glucose sensor, including a receiver, a medicament delivery device, a controller module, and optionally a single point glucose monitor. Integration may be manual, semi-automated and/or fully automated.

### Summary

A system for a method of treating hypoglycemia according to independent claim 1 is provided.

Another system of treating hypoglycemia includes means to detect a hypoglycemic condition using a continuous blood glucose monitor that monitors blood glucose levels of a patient on a continuous basis over a period of time. Further means of the system provides an alarm alerting of the hypoglycemic condition. Glucagon is delivered to the patient using an alarm and injector apparatus of the system comprising a glucagon delivery mechanism for delivering glucagon to the patient.

A further system of treating hypoglycemia includes means to detect a hypoglycemic condition using a continuous blood glucose monitor that monitors blood glucose levels of a patient on a continuous basis over a period of time. Further means provides a first alarm signal to an alarm and injector apparatus worn by the patient if the hypoglycemic condition is above a predetermined blood glucose level, or a second alarm signal to the alarm and injector apparatus worn by the patient if the hypoglycemic condition is below the predetermined blood glucose level. An alarm is provided using the alarm and injector apparatus is configured to provide an alarm in response to one or both of the first alarm signal and the second alarm signal. The alarm and injector apparatus comprises a glucagon delivery mechanism for delivering glucagon to the patient.

Another system for treating hypoglycemia includes a continuous blood glucose monitor that monitors blood glucose levels of a patient on a continuous basis over a period of time. An alarm and injector apparatus is configured to be worn by the patient and includes a glucagon delivery mechanism for automatically delivering glucagon to the patient if a hypoglycemic condition is detected using the continuous blood glucose monitor.

### Brief description of the Drawings

The following detailed description of the embodiments of the present invention can be best understood when read in conjunction with the following drawings, where like structure is indicated with like reference numerals, and in which:
FIG. 1 illustrates an embodiment of a system and method for detecting a patient's low blood glucose level and alerting the patient when the low blood glucose level is detected;
FIG. 2 is a schematic illustration of an embodiment of an alarm and injector device for use in the system and method of FIG. 1;
FIG. 3 is a schematic illustration of an embodiment of a blood glucose meter for use in the system and method of FIG. 1;
FIG. 4 illustrates an embodiment of an alarm and detector device for use in the system and method of FIG. 1;
FIG. 5 illustrates an embodiment of an infusion device for use with the alarm and detector device of FIG. 4;
FIG. 6 illustrates an embodiment of a method for detection and treatment of a hypoglycemic condition;
FIG. 7 illustrates another embodiment of a method for detection and treatment of a hypoglycemic condition; and
FIG. 8 illustrates an embodiment of a manually initiated injection method.

### Detailed Description

The following description of the preferred embodiment is merely exemplary in nature and is in no way intended to limit the invention or its application or uses.

Embodiments described herein generally relate to systems and methods that detect a patient's low blood glucose level and alert the patient (or caregiver) when the low blood glucose level is detected. As used herein, the term "patient" refers to a person who is under medical care or treatment. In some instances, the patient may be unaware of the condition. As one example, which will be described in greater detail below, the systems and methods may detect a low blood glucose condition before the condition reaches a dangerous level and provide an alarm stimulus to the patient (or caregiver). If the patient (or caregiver) does not respond to the alarm stimulus within a reasonable time (e.g., the patient is sleeping), the systems and methods may provide an automatic glucagon bolus to raise the blood glucose level.

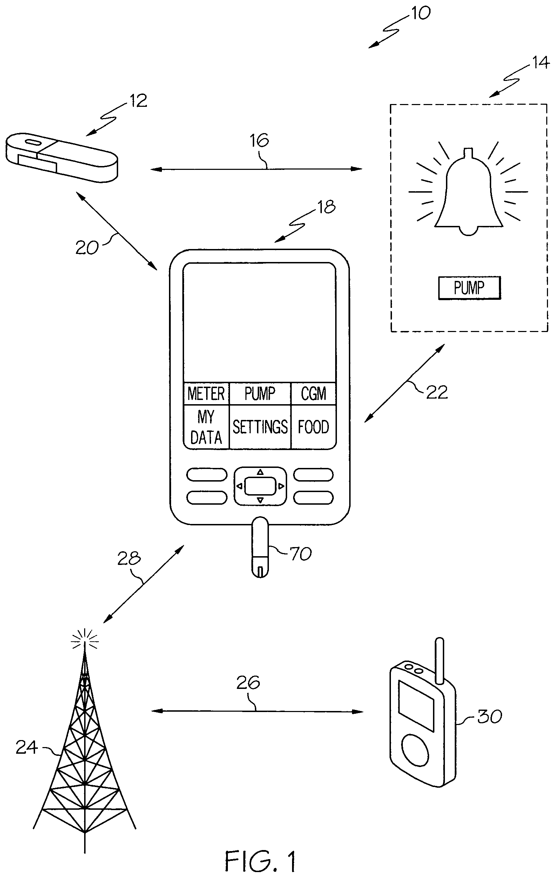

Referring to FIG. 1, a hypoglycemic alarm and treatment system 10 includes a continuous glucose monitor 12 and an alarm and injector apparatus 14 that is capable of communicating with the continuous glucose monitor 12 directly, as represented by arrow 16, and/or indirectly, for example, through a blood glucose meter 18 (or other portable device, such as a mobile phone), as represented by arrows 20 and 22. Communication between the various devices may be accomplished through any suitable wired and/or wireless connection, such as IR, Bluetooth, WLAN, other radio frequencies, the Internet, LAN etc. In some embodiments, a relay transmitter 24 may be provided for receiving signals indicative of blood glucose level from the blood glucose meter 18, as represented by arrow 28. The transmitter 24 may receive signals from any of the continuous glucose monitor 12, the alarm and injector apparatus 14 and/or the blood glucose meter 18. The signals may be transmitted as represented by arrow 26 from the relay transmitter 24 (e.g., a bedside relay transmitter) to a receiver 30 (e.g., a portable receiver), which may include an alarm, for example, for alerting a caregiver of the low blood glucose condition. In a preferable embodiment of the inventive system 10 the alarm and injector apparatus 14 is configured to automatically deliver glucagon to the patient if a hypoglycemic condition is detected using the continuous blood glucose monitor 12.

The continuous glucose monitor 12 may be used to obtain time-resolved data and may communicate the data to the blood glucose meter 18 to identify fluctuations and trends that may otherwise go unnoticed with spot monitoring of blood glucose levels and standard HbA1c tests, such as low overnight glucose levels, high blood glucose levels between meals, early morning spikes in blood glucose levels, and how diet and physical activity affect blood glucose along with the effect of therapy changes. While the continuous blood glucose monitor 12 may communicate such data to the blood glucose meter 18 for processing such data, the continuous blood glucose monitor 12 may, itself, include a processor for identifying a low (or high) blood glucose condition and/or data may be provided to devices other than the blood glucose meter 18, such as a handheld computing device, cellular phone, or other computing device. An example of a continuous glucose monitor 12 is described in U.S. Patent No. 7,389,133, which is hereby incorporated by reference. An example of a continuous blood glucose monitor working with a mobile device and blood glucose meter is described in pending U.S. Patent Application Serial No. 12/579,714, titled "Systems and Method for Providing Guidance in Administration of a Medicine," filed October 15, 2009, the details of which are incorporated by reference as if fully set forth herein.

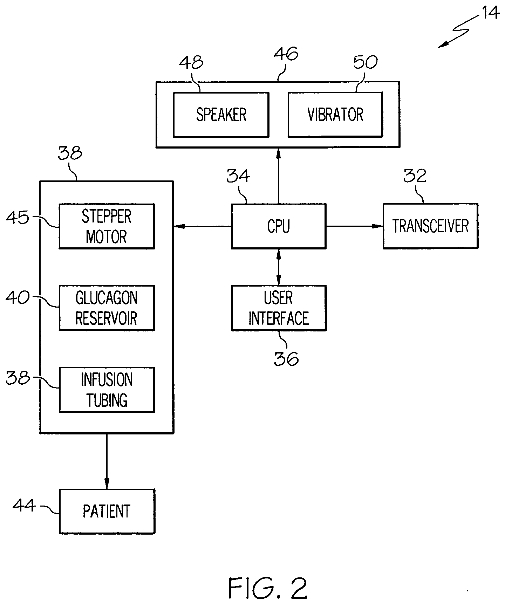

Referring now to FIG. 2, the alarm and injector apparatus 14 may be an externally worn infusion device that is configured to deliver glucagon to the patient. The alarm and injector apparatus 14 may include a power supply, a transceiver 32 (e.g., configured for receiving signals from the continuous glucose monitor 12 and/or the blood glucose meter 18), a central processing unit 34 for processing the signals as an example of an controller, a user interface 36 configured to allow input by the patient, caregiver, etc., and a delivery system 38 as a glucagon delivery mechanism that may be controlled by the central processing unit 34 and used to propel glucagon from a glucagon reservoir 40 through infusion tubing 42 to the patient 44. A stepper motor 45 or other device (e.g., a spring) may be used to propel the glucagon from the glucagon reservoir 40. An alarm system 46 may also be controlled by the central processing unit 34. The alarm system 46 may include, for example, a sound-producing device 48 for generating an audible alarm and a vibrating device 50 for producing a tactile alarm.

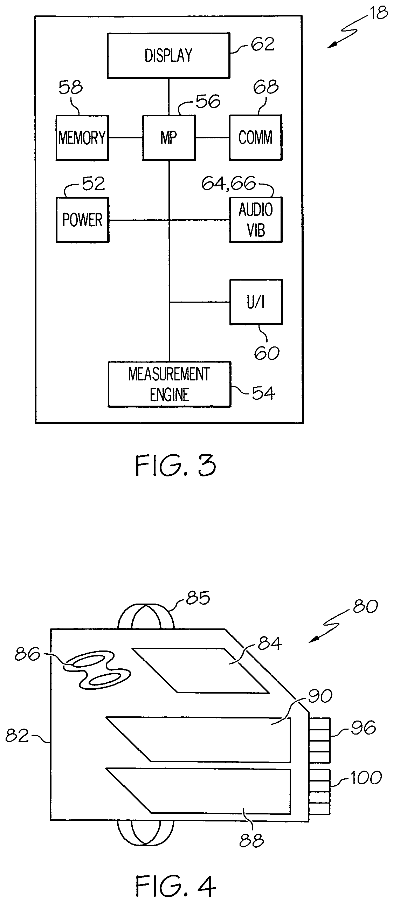

The glucose meter 18 may be a self-monitoring blood glucose meter. An example of a blood glucose meter is an Accu-Chek® Aviva described in the booklet "AccuChek® Aviva Blood Glucose Meter Owner's Booklet" (2007), portions of which are disclosed in U.S. Patent No. 6,645,368, the details of which are hereby incorporated by reference in their entirety. Referring briefly to FIG. 3, the glucose meter 18 may include a power supply 52, measurement engine 54, microprocessor 56, memory 58, user interface 60 and display 62. Some embodiments of the glucose meter 18 may include features such as diabetes software run by processor 56, an audio device 64, a vibrator device 66 and communications module 68, e.g., for communication with the alarm and injector apparatus 14.

To test glucose with the blood glucose meter 18, a small sample of blood may be placed on a disposable test strip 70 (FIG. 1). The portable hand-held blood glucose meter 18 may include a strip port that receives the disposable test strip 70. The test strip 70 may be coated with chemicals (glucose oxidase, dehydrogenase, or hexokinase) that combine with glucose in blood. The blood glucose meter 18 may then measure concentration of glucose in the blood sample. The blood glucose meter 18 may then display the glucose concentration as a number (or glucose measurement value). This glucose measurement value may be used in selecting whether to administer a long-acting insulin, a short-acting insulin or both and the dosage. In some embodiments, the glucose measurement value may be communicated to the alarm and injector apparatus 14 and used to determine (e.g., using central processing unit 34) whether a low blood glucose condition is present. In another embodiment, the blood glucose meter 18 may, itself, determine a low blood glucose condition exists and communicate such to the alarm and injector apparatus 14.

The level of blood glucose low enough to define hypoglycemia may be different for different people, in different circumstances, and for different purposes. Many healthy adults, for example, maintain fasting glucose levels above 70 mg/dL (3.9 mmol/L), and develop symptoms of hypoglycemia when the glucose falls below 55 mg/dL (3 mmol/L). Thus, the particular blood glucose level for which an alarm (and/or glucagon injection) may be given may depend on the particular patient and may be set only after a medical evaluation of the patient by a physician. Additionally, the alarm and/or injection settings may change over time. In some embodiments, the alarm and injector apparatus 14 may include logic run by the central processing unit 34 that provides an audible and/or tactile feedback alarm when it is determined (e.g., by the continuous blood glucose monitor 12, by the blood glucose meter 18 and/or by the alarm and injector apparatus 14) that blood glucose levels are at or below about 85 mg/dL, such as at or below about 70 mg/dL, such as at or below about 60 mg/dL, such as at or below about 55 mg/dL, such as at or below about 50 mg/dL, such as at or below about 45 mg/dL, such as at or below about 40 mg/dL. In some embodiments, the audible signal and/or tactile feedback may change depending on blood glucose levels determined. For example, the audible signal may change in pitch, intensity, etc. and the tactile feedback may change in frequency, intensity, etc. depending on a change in blood glucose level.

Referring to FIG. 4, an exemplary embodiment of an alarm and injector apparatus 80 may include a housing 82 within which many, if not all, of the components shown and described by FIG. 2 may be housed. The alarm and injector apparatus 80 may include a strap 85 and/or any other suitable mechanism for allowing the patient to wear the alarm and injector apparatus 80 on the patient's body. In the illustrated embodiment, the alarm and injector apparatus may include a display 84, user input 86, a glucagon reservoir 88 and an insulin reservoir 90, thereby providing a dual pump implementation of the alarm and injector apparatus 80. The display 84 may be a touch screen or other type of display. The user input 86 functions as an input mechanism and may include a touch pad or keyboard which may provide a QWERTY layout in one embodiment, a AZERTY or QWERTZ or a simple switch. In some embodiments, the alarm and injector apparatus 80 may include only a single pump with a single, glucagon reservoir. In some embodiments, one or both of the medications from the reservoirs 88 and 90 may be dispensed in a metered fashion, for example, using a stepper motor. In another embodiment, a spring or other delivery device may be used to deliver the entirety of the reservoirs using, for example, a spring as a single bolus. So different kinds of glucagon delivery mechanisms can be implemented in the alarm and injector apparatus 80.

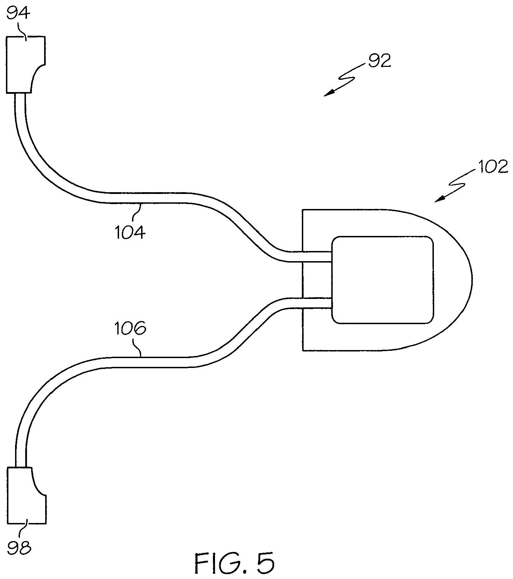

Referring to FIG. 5, an infusion device 92 may be used to deliver the insulin and/or glucagon to a transcutaneous site in the patient. The infusion device 92 may include a first connector 94, e.g., for connecting to port 96 of the alarm and injector apparatus 80 (FIG. 4) and a second connector 98, e.g., for connecting to port 100 of the alarm and injector apparatus 80. In some embodiments, a single skin interface device 102 (e.g., a patch) may receive both cannulas 104 and 106 connected to their respective connectors 94 and 98. In another embodiment, each cannula 104 and 106 may run to a separate skin interface device. Each cannula 104 and 106 may be in communication with one or more hollow projections (e.g., a needle) carried by the skin interface device 102 capable of penetrating the skin once the skin interface device 102 is positioned on the skin. The projections are used to administer the glucagon and/or insulin to the patient.

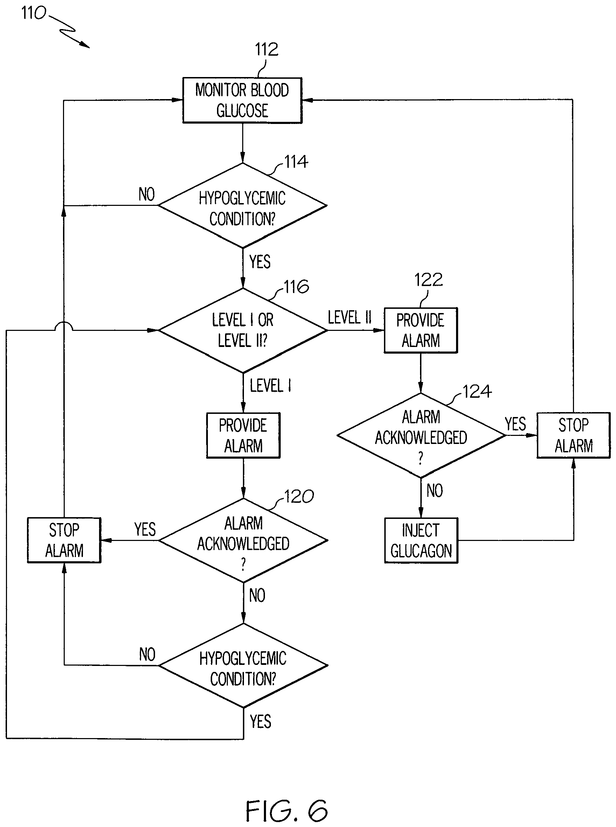

As indicated above, the hypoglycemic alarm and treatment system 10 may detect a low blood glucose condition before the condition reaches a dangerous level and provide an alarm stimulus to the patient. If the patient does not respond to the alarm stimulus within a reasonable time while the blood glucose level continues to decrease (e.g., while the patient is sleeping), the systems and methods may provide an automatic glucagon bolus to the patient to raise the blood glucose. FIG. 6 illustrates an exemplary method 110 for detection and treatment of a hypoglycemic condition using the alarm and treatment system 10. At step 112, the continuous blood glucose monitor 12 monitors the patient's blood glucose level. At step 114, the continuous blood glucose monitor 12 determines whether a hypoglycemic condition exists. In some embodiments, the continuous blood glucose monitor 12 may, itself, determine whether the hypoglycemic condition exits. However, the blood glucose meter 18 and/or the alarm and injector apparatus 14 may determine whether the hypoglycemic condition exists based on blood glucose information from the continuous blood glucose monitor 12. If no hypoglycemic condition exists, the continuous blood glucose monitor 12 continues to monitor blood glucose. If a hypoglycemic condition exists, it is determined whether a Level I (minor hypoglycemic condition) or a Level II (major hypoglycemic condition) hypoglycemic condition exits at step 116. Any of the blood glucose monitor 12, the blood glucose meter 18 and/or the alarm and injector apparatus 14 may determine whether a Level I or a Level II hypoglycemic condition exists based on the blood glucose information from the continuous blood glucose monitor 12.

In a Level I hypoglycemic condition, a low blood glucose alarm (e.g., audible and/or vibration) may be provided (e.g., by the continuous blood glucose monitor 12, the blood glucose meter 18, the alarm and injector apparatus 14 and/or the receiver 30) indicating a low blood glucose condition at step 118. The blood glucose level for triggering the alarm may be configurable (e.g., using user interface 36 on the alarm and injector apparatus 14, on the blood glucose meter 18 and/or on the continuous blood glucose monitor 12) and may be in the range of about 70 to about 85 mg/mL. Glucagon may not be delivered during this Level I hypoglycemic condition. The alarm may continue until acknowledged by the patient or caregiver at step 120 using one or more of the user inputs of the alarm and injector apparatus 14, on the blood glucose meter 18 and/or on the continuous blood glucose monitor 12. If the alarm is acknowledged, the alarm may be discontinued and the continuous blood glucose monitor 12 continues to monitor the patient's blood glucose levels. If the alarm is not acknowledged, it is again determined whether a Level I or a Level II hypoglycemic condition exits.

In many instances, if the alarm is not acknowledged, corrective action is not being taken to correct blood glucose levels. In some cases, blood glucose levels of the patient may continue to decline. If at step 116 it is determined that a Level II hypoglycemic condition exists, another low blood glucose alarm (e.g., audible and/or vibration) may be provided (e.g., by the continuous blood glucose monitor 12, the blood glucose meter 18, the alarm and injector apparatus 14 and/or the receiver 30) indicating the lower blood glucose condition at step 122. The other low blood glucose alarm may be different than the first blood glucose alarm associated with Level I to indicate the more severe Level II condition. The Level II settings may be provided by a medical doctor, for example, after patient testing and may be below about 70 mg/mL. Security systems may be provided such that Level II settings may be set only by medical personnel and not the patient. The low blood glucose alarm may continue until acknowledged by the patient or caregiver at step 124 using one or more of the user inputs of the alarm and injector apparatus 14, on the blood glucose meter 18 and/or on the continuous blood glucose monitor 12. If the low blood glucose alarm is acknowledged at step 124, the patient or caretaker can administer a glucagon bolus and the alarm and injector apparatus 14 will not automatically inject the patient with glucagon. If the low blood glucose alarm is not acknowledged at step 124 within a preselected time period (e.g., 10 minutes or other configurable time period, for example, as determined by central processing unit 34), automatic delivery of the glucagon begins at step 126. When the bolus of glucagon is delivered, the display 84 may display a message, such as "glucagon delivered" that remains on display until acknowledged by the patient or caregiver.

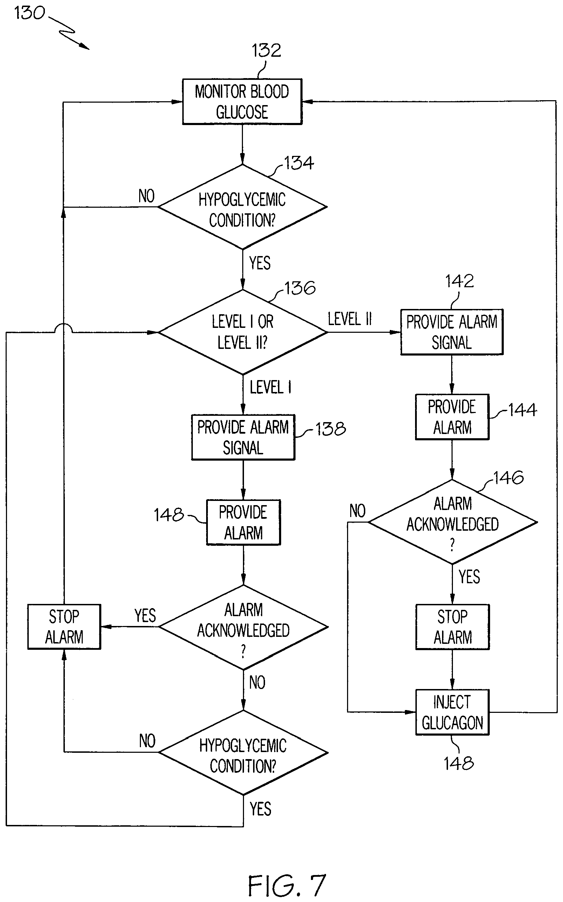

Referring to FIG. 7, another exemplary method 130 for directing delivery of glucagon is provided. This method may be performed, for example, when the patient is unconscious and/or experiencing a hypoglycemic seizure or is otherwise unaware of the hypoglycemic condition and thus fails to initiate delivery of glucagon. At step 132, the continuous blood glucose monitor 12 monitors the patient's blood glucose level. At step 134, the continuous blood glucose monitor 12 determines whether a hypoglycemic condition exists. In some embodiments, the continuous blood glucose monitor 12 may, itself, determine whether the hypoglycemic condition exits. However, the blood glucose meter 18 and/or the alarm and injector apparatus 14 may determine whether the hypoglycemic condition exists based on blood glucose information from the continuous blood glucose monitor 12. If no hypoglycemic condition exists, the continuous blood glucose monitor 12 continues to monitor blood glucose. If a hypoglycemic condition exists, it is determined whether a Level I (minor hypoglycemic condition) or a Level II (major hypoglycemic condition) hypoglycemic condition exits at step 136. Any of the blood glucose monitor 12, the blood glucose meter 18 and/or the alarm and injector apparatus 14 may determine whether a Level I or a Level II hypoglycemic condition exists based on the blood glucose information from the continuous blood glucose monitor 12.

If a Level I hypoglycemic condition exists, an alarm signal may be sent from any one of the continuous blood glucose monitor 12, alarm and injector apparatus 14 and/or blood glucose meter 18 to the receiver 30 via the transmitter relay 24 (FIG. 1) at step 138. In response to the alarm signal, the receiver 30 may provide an alarm (e.g., sounds, vibrations, etc.) to the caregiver, for example, in a different room at step 140. The type of the alarm my be configured to indicate to the caregiver of a severely low blood glucose level (e.g., Level I or Level II). A user interface may be provided on the receiver 30 that allows the caregiver to stop the alarm. If the alarm is acknowledged, the alarm may be discontinued and the continuous blood glucose monitor 12 continues to monitor the patient's blood glucose levels. If the alarm is not acknowledged, it is again determined whether a Level I or a Level II hypoglycemic condition exits.

If at step 136 it is determined that a Level II hypoglycemic condition exists, another alarm signal may be sent from any one of the continuous blood glucose monitor 12, alarm and injector apparatus 14 and/or blood glucose meter 18 to the receiver 30 via the transmitter relay 28 (FIG. 1) at step 142. In response to the alarm signal, the receiver 30 may provide an alarm (e.g., sounds, vibrations, etc.) to the caregiver, for example, in a different room at step 144. The other low blood glucose alarm may be different than the first blood glucose alarm associated with Level I to indicate the more severe Level II condition. The low blood glucose alarm may continue until acknowledged by the patient or caregiver at step 146. If the low blood glucose alarm is acknowledged at step, the caretaker can administer a glucagon bolus at step 148. If the alarm is not acknowledged within a period of time (e.g., 10 minutes), the glucagon bolus may be administered automatically in a fashion similar to that described above.

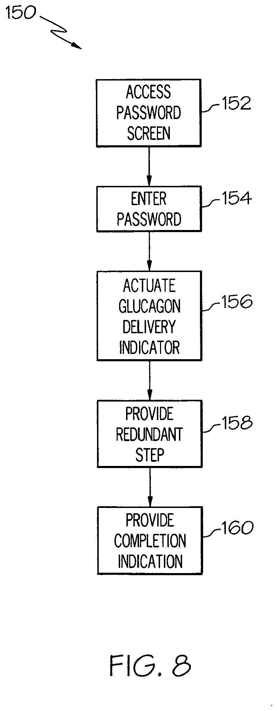

Referring to FIG. 8, an exemplary manually initiated injection method 150 is shown. At step 152, a password screen may be accessed by the caregiver on the alarm and injector apparatus 14 and a password may be entered at step 154 to access a glucagon bolus screen. A glucagon delivery indicator may be actuated (e.g., a button may be pressed) at step 156. A redundant confirmation may be required at step 158. For example, the button may need to be pressed again and held for a period of time (e.g., two seconds) or an "are you sure?" or similar prompt may be generated. Any suitable redundant confirmation may be utilized that reduces the probability of an unintended delivery of glucagon to the patient. A stop control may be provided to allow the caregiver to halt injection of glucagon. A screen may be provided that indicates completion of glucagon bolus delivery at step 160.

In some embodiments, the receiver 30 may allow for monitoring of the patient's condition remotely. For example, the receiver 30 may include a processor and software that provides information regarding the patient's blood glucose levels. In some embodiments, the receiver 30 may allow for remote operation (e.g., injection) of the alarm and injector apparatus 14.

The systems and methods described above may detect a low blood glucose condition before the condition reaches a dangerous level and provide an alarm stimulus to the patient and/or caregiver. If the patient or caregiver does not respond to the alarm stimulus within a reasonable time (e.g., the patient is sleeping), the systems and methods may provide an automatic glucagon bolus to raise the blood glucose level. The systems and methods may also allow for manual initiation of the glucagon bolus, for example, in response to the alarm stimulus.

## Claims

1. A system (10) for a method of treating hypoglycemia, wherein the system (10) comprises means to:
- detect a hypoglycemic condition using a continuous blood glucose monitor (12) of the system (10) that monitors blood glucose levels of a patient on a continuous basis over a period of time;
- determine whether the hypoglycemic condition is either a minor hypoglycemic condition, corresponding to the blood glucose level of the patient being below a first configurable blood glucose level, or a major hypoglycemic condition, corresponding to the blood glucose level of the patient being below a second configurable blood glucose level, the second configurable blood glucose level being lower than the first configurable blood glucose level;
- provide a first alarm alerting of the hypoglycemic condition if the hypoglycemic condition is a minor hypoglycemic condition;
- provide a second alarm alerting of the hypoglycemic condition if the hypoglycemic condition is a major hypoglycemic condition; and
- deliver insulin and glucagon to the patient using a dual pump implementation of an alarm and injector apparatus (14, 80) of the system (10) comprising
- an insulin reservoir (90),
- a glucagon reservoir (88),
- an insulin delivery mechanism for use in delivering insulin from the insulin reservoir (90) to the patient, and
- a glucagon delivery mechanism (38) for delivering glucagon from the glucagon reservoir (88) to the patient,
and configured to automatically deliver glucagon if after a predetermined time period the second alarm is not acknowledged.

2. The system (10) of claim 1 further comprising means to provide an alarm signal to the alarm and injector apparatus (14, 80) in response to detecting the hypoglycemic condition.

3. The system (10) of claim 1 or 2, comprising means to provide the first and second alarm signal from the continuous blood glucose monitor (12) to the alarm and injector apparatus (14, 80).

4. The system (10) of any of the claims 1 to 3, wherein the alarm and injector apparatus (14, 80) includes a controller controlling an alarm mechanism providing the first and second alarm, and wherein the second alarm is different from the first alarm.

5. The system (10) of claim 4, comprising an input mechanism, and the controller comprises means to, after the step of providing the alarm, look for a patient-initiated input signal provided using the input mechanism within the predetermined time period.

6. The system (10) of claim 5, wherein the controller comprises means to, if the patient-initiated input signal is not received by the controller within the predetermined time period, actuate the glucagon delivery mechanism.

7. The system (10) of any of the claims 1 to 6, wherein the alarm and injector apparatus (14, 80) is configured to be worn by the patient (44).

8. The system (10) of any of the claims 1 to 7, wherein the continuous blood glucose monitor (12) comprises a transmitter for transmitting an alarm signal to the alarm and injector apparatus (14, 80) if a hypoglycemic condition is detected.

9. The system (10) of any of the claims 1 to 8, further comprising a blood glucose meter (18) configured to receive blood glucose information from the continuous blood glucose monitor (12).

10. The system (10) of claim 9, wherein the blood glucose meter (18) comprises a transmitter for transmitting an alarm signal to the alarm and injector apparatus (14, 80) if a hypoglycemic condition is detected.

11. The system (10) of any of the claims 1 to 10, wherein one or both medications from the reservoirs (88, 90) are delivered either in a metered fashion or in its entirety.

## Patentansprüche

1. System (10) für ein Verfahren für die Behandlung von Hypoglykämie, wobei das System (10) Mittel umfasst zum:
- Erfassen eines hypoglykämischen Zustands durch Verwenden eines kontinuierlichen Blutglukosemonitors (12) des Systems (10), der Blutglukosespiegel eines Patienten auf kontinuierlicher Basis über eine Zeitspanne verfolgt;
- Bestimmen, ob der hypoglykämische Zustand entweder ein unbedeutender hypoglykämischer Zustand, der dem Blutglukosespiegel des Patienten entspricht, der unter einem ersten konfigurierbaren Blutglukosespiegel liegt, oder ein bedeutender hypoglykämischer Zustand ist, der dem Blutglukosespiegel des Patienten entspricht, der unter einem zweiten konfigurierbaren Blutglukosespiegel liegt, wobei der zweite konfigurierbare Blutglukosespiegel niedriger als der erste konfigurierbare Blutglukosespiegel ist;
- Bereitstellen eines ersten Alarms, der auf den hypoglykämischen Zustand aufmerksam macht, wenn der hypoglykämische Zustand ein unbedeutender hypoglykämischer Zustand ist;
- Bereitstellen eines zweiten Alarms, der auf den hypoglykämischen Zustand aufmerksam macht, wenn der hypoglykämische Zustand ein bedeutender hypoglykämischer Zustand ist; und
- Verabreichen von Insulin und Glucagon an den Patienten mithilfe einer Dualpumpenausführung einer Alarm- und Injektorvorrichtung (14, 80) des Systems (10) umfassend
- ein Jnsulinreservoir (90),
- ein Glucagonreservoir (88),
- einen Insulinverabreichungsmechanismus zur Verwendung zum Verabreichen von Insulin aus einem Insulinreservoir (90) an den Patienten,
- einen Glucagonverabreichungsmechanismus (38) zum Verabreichen von Glucagon von dem Glucagonreservoir (88) an den Patienten,
und konfiguriert, um automatisch Glucagon zu verabreichen, wenn nach einer vorbestimmten Zeitspanne der zweite Alarm nicht bestätigt wird.

2. System (10) nach Anspruch 1, ferner umfassend Mittel zum Bereitstellen eines Alarmsignals an die Alarm- und Injektorvorrichtung (14, 80) als Reaktion auf das Erfassen des hypoglykämischen Zustands.

3. System (10) nach Anspruch 1 oder 2, umfassend Mittel zum Bereitstellen des ersten und zweiten Alarmsignals von dem kontinuierlichen Blutglukosemonitor (12) zu der Alarm- und Injektorvorrichtung (14, 80).

4. System (10) nach einem der Ansprüche 1 bis 3, wobei die Alarm- und Injektorvorrichtung (14, 80) eine Steuerung umfasst, die einen Alarmmechanismus steuert, die den ersten und zweiten Alarm bereitstellt und wobei der zweite Alarm von dem ersten Alarm verschieden ist.

5. System (10) nach Anspruch 4, umfassend einen Eingabemechanismus wobei und die Steuerung Mittel umfasst zum Ausschauhalten, nach dem Schritt des Bereitstellens des Alarms, nach einem patientenausgelösten Eingabesignal, das unter Verwendung des Eingabemechanismus innerhalb der vorbestimmten Zeitspanne bereitgestellt wird.

6. System (10) nach Anspruch 5, wobei die Steuerung Mittel umfasst zum Auslösen, wenn das patientenausgelöste Eingabesignal nicht von der Steuerung innerhalb der vorbestimmten Zeitspanne erhalten wird, des Glucagonverabreichungsmechanismus.

7. System (10) nach einem der Ansprüche 1 bis 6, wobei die Alarm- und Injektorvorrichtung (14, 80) konfiguriert ist, vom Patienten (44) getragen zu werden.

8. System (10) nach einem der Ansprüche 1 bis 7, wobei der kontinuierliche Blutglukosemonitor (12) einen Transmitter zum Übertragen eines Alarmsignals zu der Alarm- und Injektorvorrichtung (14, 80) umfasst, wenn ein hypoglykämischer Zustand erfasst wird.

9. System (10) nach einem der Ansprüche 1 bis 8, ferner umfassend eine Blutglukosemessvorrichtung (18), die zum Empfangen von Blutglukoseinformationen von dem kontinuierlichen Blutglukosemonitor (12) konfiguriert ist.

10. System (10) nach Anspruch 9, wobei die Blutglukosemessvorrichtung (18) einen Transmitter zum Übertragen eines Alarmsignals zu der Alarm- und Injektorvorrichtung (14, 80) umfasst, wenn ein hypoglykämischer Zustand erfasst wird.

11. System (10) nach einem der Ansprüche 1 bis 10, wobei ein oder beide Medikamente aus den Reservoirs (88, 90) entweder auf dosierte Weise oder in ihrer Gesamtheit verabreicht werden.

## Revendications

1. Système (10) pour un procédé de traitement de l'hypoglycémie, dans lequel le système (10) comprend un moyen de :
- détection d'un état d'hypoglycémie à l'aide d'un moniteur de glycémie continu (12) du système (10) qui surveille les niveaux de glycémie d'un patient sur une base continue pendant une période de temps ;
- détermination du fait que l'état d'hypoglycémie est soit un état d'hypoglycémie mineure, correspondant à un niveau de glycémie du patient inférieur à un premier niveau de glycémie configurable, soit un état d'hypoglycémie majeure, correspondant à un niveau de glycémie du patient inférieur à un second niveau de glycémie configurable, le second niveau de glycémie configurable étant inférieur au premier niveau de glycémie configurable ;
- fourniture d'une première alarme avertissant de l'état d'hypoglycémie si l'état d'hypoglycémie est un état d'hypoglycémie mineure ;
- fourniture d'une seconde alarme avertissant de l'état d'hypoglycémie si l'état d'hypoglycémie est un état d'hypoglycémie majeure ; et
- administration d'insuline et de glucagon au patient en utilisant une implémentation de pompe double d'un appareil d'alarme et d'injection (14, 80) du système (10) comprenant
- un réservoir d'insuline (90),
- un réservoir de glucagon (88),
- un mécanisme d'administration d'insuline pour son utilisation dans l'administration d'insuline du réservoir d'insuline (90) au patient, et
- un mécanisme d'administration de glucagon (38) pour administrer du glucagon du réservoir de glucagon (88) au patient,
et configuré pour automatiquement administrer le glucagon si après une période de temps prédéterminée la seconde alarme n'est pas acquittée.

2. Système (10) selon la revendication 1 comprenant en outre un moyen de fourniture d'un signal d'alarme à l'appareil d'alarme et d'injection (14, 80) en réponse à la détection de l'état d'hypoglycémie.

3. Système (10) selon la revendication 1 ou 2, comprenant un moyen de fourniture des premier et second signaux d'alarme du moniteur de glycémie continu (12) à l'appareil d'alarme et d'injection (14, 80).

4. Système (10) selon l'une quelconque des revendications 1 à 3, dans lequel l'appareil d'alarme et d'injection (14, 80) inclut un dispositif de commande commandant un mécanisme d'alarme fournissant les première et seconde alarmes, et dans lequel la seconde alarme est différente de la première alarme.

5. Système (10) selon la revendication 4, comprenant un mécanisme d'entrée, et le dispositif de commande comprend un moyen de recherche, après l'étape de fourniture de l'alarme, d'un signal d'entrée initié par le patient fourni en utilisant le mécanisme d'entrée au cours de la période de temps prédéterminée.

6. Système (10) selon la revendication 5, dans lequel le dispositif de commande comprend un moyen d'actionnement, si le signal d'entrée initié par le patient n'est pas reçu par le dispositif de commande au cours de la période de temps prédéterminée, du mécanisme d'administration de glucagon.

7. Système (10) selon l'une quelconque des revendications 1 à 6, dans lequel l'appareil d'alarme et d'injection (14, 80) est configuré pour être porté par le patient (44).

8. Système (10) selon l'une quelconque des revendications 1 à 7, dans lequel le moniteur de glycémie continu (12) comprend un transmetteur pour transmettre un signal d'alarme à l'appareil d'alarme et d'injection (14, 80) si un état d'hypoglycémie est détecté.

9. Système (10) selon l'une quelconque des revendications 1 à 8, comprenant en outre un glucomètre (18) configuré pour recevoir des informations de glycémie du moniteur de glycémie continu (12).

10. Système (10) selon la revendication 9, dans lequel le glucomètre (18) comprend un transmetteur pour transmettre un signal d'alarme à l'appareil d'alarme et d'injection (14, 80) si un état d'hypoglycémie est détecté.

11. Système (10) selon l'une quelconque des revendications 1 à 10, dans lequel l'un des médicaments des réservoirs (88, 90) ou les deux sont administrés par dose ou dans leur totalité.
